(19) Europäisches Patentamt
European Patent Office
Office européen des brevets

(11) Veröffentlichungsnummer: **0 252 378 B1**

(12) # EUROPÄISCHE PATENTSCHRIFT

(45) Veröffentlichungstag der Patentschrift: **27.09.89**

(51) Int. Cl.[4]: **C07C 47/02**, C07C 47/21, C07C 45/73

(21) Anmeldenummer: **87109170.8**

(22) Anmeldetag: **26.06.87**

(54) **Neue aliphatische Aldehyde, Verhahren zu deren Herstellung und deren Verwendung als Riechstoffe.**

(30) Priorität: **05.07.86 DE 3622600**

(43) Veröffentlichungstag der Anmeldung: **13.01.88 Patentblatt 88/2**

(45) Bekanntmachung des Hinweises auf die Patenterteilung: **27.09.89 Patentblatt 89/39**

(84) Benannte Vertragsstaaten: **CH DE ES FR GB LI NL**

(56) Entgegenhaltungen:
**DE-A- 2 229 269**
**DE-A- 2 351 057**
**DE-C- 1 149 344**
**FR-A- 2 077 344**
**US-A- 4 240 985**

**CHEMICAL ABSTRACTS, Band 44, Nr. 7, 10. April 1950, Spalte 2911h, Columbus, Ohio, US; W.M. BRUNER "3,5,5-Trimethylhexanol and its derivatives"**

(73) Patentinhaber: **BASF Aktiengesellschaft, Carl-Bosch-Strasse 38, D-6700 Ludwigshafen(DE)**

(72) Erfinder: **Gramlich, Walter, Dr., Auf der Hoehe 11, D-6803 Edingen-Neckarhausen(DE)**
Erfinder: **Siegel, Hardo, Dr., Hans-Purrmann-Allee 25, D-6720 Speyer(DE)**

**EP 0 252 378 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft aliphatische Aldehyde der allgemeinen Formel I

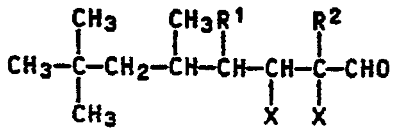

(I),

in der

$R^1$ für Wasserstoff oder eine Methylgruppe steht,

$R^2$ für einen geradkettigen oder verzweigten Alkylrest mit 1 bis 4 C-Atomen, vorzugsweise eine Methyl- oder Ethylgruppe, steht und die beiden X jeweils für Wasserstoff stehen oder zusammen eine weitere CC-Bindung bedeuten,

insbesondere

2,5,7,7-Tetramethyl-octanal, 2,4,5,7,7-Pentamethyl-octanal, 2-Ethyl-5,7,7-trimethyl-octanal und 2,5,7,7-Tetramethyl-2-octen-1-al, deren Herstellung und Verwendung als Riechstoffe.

Wegen der im allgemeinen von vielen unwägbaren Faktoren abhängigen unsicheren Verfügbarkeit vieler natürlicher Riechstoffkomponenten sowie der notwendigen Anpassung an wechselnde modische Geschmacksrichtungen hat die Riechstoffindustrie ständig Bedarf an neuen Riechstoffen, die entweder allein oder in Form von Kompositionen wertvolle Parfüms mit interessanten Duftnoten darstellen. Darüber hinaus ist ein ständig wachsender Trend zur Parfümierung von Produkten des täglichen Bedarfs, wie Kosmetika und technischen Artikeln wie Leimen, Wasch- und Reinigungsmitteln, für Raumsprays und anderem festzustellen.

Da aufgrund wenig bekannter Zusammenhänge zwischen Struktur und olfaktorischen Eigenschaften eine gezielte Synthese von Riechstoffen mit gewünschter Geruchsqualität nicht möglich ist, besteht die Aufgabe, solche Verbindungen aufzufinden, die wertvolle Riechstoffeigenschaften aufweisen, d.h. beispielsweise neue Geruchsnoten aufweisen oder teure oder schwer zugängliche Riechstoffe ersetzen können und außerdem aus gut verfügbaren und wenig Umwelt-belastenden Vorprodukten hergestellt werden können.

Auf der Suche nach preiswerten, gut zugänglichen neuen Riechstoffen mit interessanten Geruchsnoten wurden die neuen aliphatischen Aldehyde der Formel I gefunden.

Es sind bereits einige Verbindungen mit einer gewissen strukturellen Beziehung zu den erfindungsgemäßen Verbindungen bekannt.

So ist beispielsweise aus US 3 531 510 ein 5,7,7-Trimethyl-octennitril mit einem an Gewürznelke oder Iris erinnernden Geruch mit erdiger, frischer und grüner Note bekannt.

Weiterhin ist aus der DE-OS 27 23 636 das 2,3,5,5-Tetramethyl-hexanal (Aldehyd TMH) mit einem frischen, grünen Geruch, der an Kräuter, Blumen und Ozon erinnert, bekannt.

Handelsübliches 3,5,5-Trimethyl-hexanal besitzt einen starken Grün-Charakter mit öliger sowie an grünes Gemüse erinnernder Note.

Ferner sind aus der DE-OS 3 245 047 Verbindungen der Formel

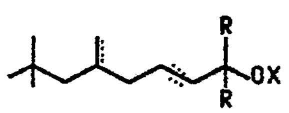

(R = H oder Alkyl

X = H oder organisches Acyl)

von denen das 5,7,7-Trimethyl-octyl-propionat mit seiner fruchtigen, birnenähnlichen Note besonders interessant erscheint.

Die erfindungsgemäßen aliphatischen Aldehyde der Formel I weisen wertvolle Duftstoffeigenschaften auf, die sich von den oben beschriebenen Verbindungen sehr deutlich unterscheiden. Beispielsweise weist das 2,5,7,7-Tetramethyl-octanal (I; $R^1$ = H; $R^2$ = $CH_3$) eine hochinteressante, intensive Maiglöckchen-Note auf. Das ist sehr überraschend, da gemäß Beispiel 5 der oben genannten DE-OS 3 245 047 die Herstellung von 6,8,8-Trimethyl-2-nonanol durch Oxidation von 5,7,7-Trimethyl-octanol zum 5,7,7-Trimethyl-octanal und anschließende Grignard-Methylierung beschrieben wird und der als Zwischenstufe aufgetretene Aldehyd, der sich von dem oben genannten Aldehyd der Formel I nur durch das Fehlen einer Methylgruppe unterscheidet, ganz offensichtlich als olfaktorisch unbedeutend eingestuft wurde und das obwohl es die Aufgabe dieser Erfindung war, gute Riechstoffe herzustellen.

Besonders interessante Aldehyde der Formel I sind diejenigen, in denen $R^1$ für Wasserstoff oder eine Methylgruppe und $R^2$ für eine Methylgruppe oder eine Ethylgruppe stehen und die X Wasserstoff bedeuten.

Eine besonders wertvolle Substanz unter den bevorzugten Verbindungen ist das 2,5,7,7-Tetramethyl-octanal, dessen intensive und doch feine Maiglöckchen-Note besonders interessant ist und mit dieser einzigartigen Note mit keinem derzeitigen Marktprodukt verglichen werden kann.

Ebenso zeigt das 2,4,5,7,7-Pentamethyl-octanal einen interessanten Blumenduft mit einer Calmus-Note.

Das 2-Ethyl-5,7,7-trimethyl-octanal weist gleichfalls eine interessante Blumennote auf und wirkt zusätzlich noch sehr frisch.

Aufgrund ihrer interessanten Geruchseigenschaften können die neuen Aldehyde der allgemeinen Formel I vorteilhaft als Riechstoffe oder Bestandteile von Parfümölen für kosmetische oder technische Anwendungen eingesetzt werden. Sie können in Kompositionen innerhalb eines breiten Konzentrationsbereiches angewandt werden und lassen sich gut mit üblichen Parfüminhaltsstoffen und anderen Riechstoffen zu neuartigen Kompositionen kombinieren. Der Anteil der erfindungsgemäßen Verbindungen kann in Riechstoffkompositonen zwischen 1 bis 50 Gew.%, bezogen auf das Gewicht der Zusammensetzung, betragen.

Derartige Kompositionen können zur Parfümierung von kosmetischen Präparaten, wie Cremes, Lotionen, Duftwässern, Toilettenseifen, Mundpflegemitteln, Aerosolen sowie in der Extrait-Parfümerie verwendet werden. Sie können weiterhin zur Geruchsverbesserung technischer Produkte wie Reinigungs- und Waschmittel sowie Weichspüler dienen. Normalerweise werden 0,05 bis 2 Gew.% einer derartigen Komposition, bezogen auf das gesamte Produkt, eingesetzt.

Neben der Verwendung der erfindungsgemäßen Aldehyde der Formel I zur Verleihung, Verbesserung oder Modifizierung der Dufteigenschaften von Parfüms oder parfümierten Produkten betrachten wir auch als Gegenstand der Erfindung ein Verfahren zur Herstellung dieser aliphatischen Aldehyde der Formel I, das dadurch gekennzeichnet ist, daß man einen Aldehyd der allgemeinen Formel II

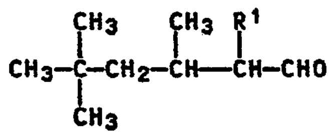

(II)

mit einem Aldehyd der allgemeinen Formel III

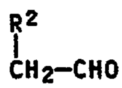

(III)

in denen $R^1$ und $R^2$ die oben angegebene Bedeutung haben in Gegenwart eines Aldolkondensationskatalysators zu dem substituierten Octenal der allgemeinen Formel I kondensiert und dieses ggf. partiell zu dem entsprechenden Octanal der Formel I hydriert bzw. reduziert.

Die Aldehyde der Formeln II und III sind handelsübliche Verbindungen. Insbesondere die Aldehyde der Formel III, wie Propionaldehyd, Butyraldehyd, Isovaleraldehyd, n-Valeraldehyd und n-Hexanal stellen bedeutende technische Zwischenprodukte dar, und stehen daher in ausreichender Menge preiswert zur Verfügung.

Die Aldolkondensation wird in an sich bekannter Weise durchgeführt, so daß sich detaillierte Ausführungen hierüber erübrigen. Sie wird bevorzugt in Gegenwart von basischen Katalysatoren durchgeführt. Als basische Katalysatoren kommen insbesondere konzentrierte Kali- oder Natronlauge zum Einsatz. Als Lösungsmittel haben sich besonders niedrige Alkohole, wie Methanol oder Ethanol bewährt. Bezüglich weiterer Einzelheiten über Aldolkondensationen verweisen wir auf Houben-Weyl, "Methoden der Organischen Chemie", Band 7/1, Seiten 76-85, besonders 76 bis 78.

Steht der Rest $R^1$ für Wasserstoff, so sind beide sowohl die Aldehyde II als auch die Aldehyde III in α-Stellung unsubstituiert, wodurch es zur Bildung eines Gemisches von drei verschiedenen Aldol-Kondensationsprodukten kommt (eine Homoaldolkondensation von Aldehyd II, eine Homoaldolkondensation von Aldehyd III sowie eine Hetero-Aldol-Kondensation).

In einer bevorzugten Variante wird der basische Katalysator mit dem Lösungsmittel (z.B. Methanol) vorgelegt und bei erhöhter Temperatur, vorzugsweise 50 bis 60°C, das Gemisch der Aldehyde II und III zugetropft.

Das Molverhältnis der Aldehyde kann je nach den Resten $R^1$ und $R^2$ variiert werden, jedoch wird im allgemeinen ein Überschuß des meist reaktionsfähigeren Aldehyds III angewendet.

Durch diese Variante kann die Homoaldol-Kondensation des Aldehyds II fast völlig verhindert werden. Die Eigenkondensationsprodukte des Aldehyds der Formel III lassen sich leicht abtrennen und stellen häufig technisch wichtige Zwischenprodukte dar, wie beispielsweise das 2-Methyl-2-pentenal (aus Propanal) oder das chemische Großprodukt 2-Ethyl-2-hexenal (aus Butanal), so daß zusätzlich Wertprodukte erhalten werden.

Die erhaltenen Octenale der Formel I können dann gewünschtenfalls nach allgemein bekannten Methoden zu den entsprechenden gesättigten Aldehyden der Formel I hydriert oder reduziert werden. Für diesen selektiven Hydrierschritt haben sich besonders Palladiumkatalysatoren auf verschiedenen Trägern, wie Kohle, Aluminiumoxid, Kieselgel oder Bauxit bewährt. Auch hierbei sind niedere Alkohole vorteilhafte Lösungsmittel. Bezüglich näherer Einzelheiten über solche selektiven Hydrierungen verweisen wir beispielsweise auf P. Rylander, "Catalytic Hydrogenation in Organic Synthesis, Academic Press 1979, Seiten 74 f.

Aufgrund der beschriebenen besonderen Riechstoffeigenschaften können die erfindungsgemäßen Aldehyde vorteilhaft als Riechstoffe oder Bestandteile von Riechstoffkompositionen und Parfümölen für kosmetische Anwendungen eingesetzt werden. Von Bedeutung ist auch, daß sie auf einfache Weise aus gut zugänglichen Ausgangsstoffen hergestellt werden können.

Beispiel 1

Herstellung von 2,5,7,7-Tetramethyl-2-octenal

In einem Reaktionskolben wurden unter Stickstoff 128 g (4 mol) Methanol und 2 g (0,05 mol) NaOH vorgelegt und unter Rückfluß zum Sieden erhitzt. Zu dieser Lösung wurde innerhalb von 10 Min. eine Mischung aus 142 g (1 mol) 3,5,5-Trimethyl-hexanal und 116 g (2 mol) Propionaldehyd zugetropft. Das erhaltene Reaktionsgemisch wurde eine Stunde (h) bei Siedetemperatur gehalten und anschließend mit einer 80 %igen wäßrigen Essigsäurelösung ein pH-Wert von 4 bis 5 eingestellt. Nach zweimaligem Waschen mit Wasser wurde die organische Phase abgetrennt und durch fraktionierte Destillation aufgearbeitet. Man erhielt als eine Vorlauf-Fraktion 32 g unumgesetztes 3,5,5-Trimethyl-hexanal sowie 113 g 2,5,7,7-Tetramethyl-2-octenal (Kp. = 108 bis 111°C/14 mbar), was einer Selektivität, bezogen auf Trimethyl-hexanal, von 81 % entspricht; $n_D^{25}$ 1.4580.
IR: 2956, 16981, 1644, 1246 cm$^{-1}$;
$^1$H-NMR: 0,85 (9H, s), 0,92 (3H, d), 1-1,28 (2H, m), 1,75 (3H, s), 1,8 (1H, m), 2,1-2,35 (2H, m), 6,53 (1H, t), 9,4 (1H, s) δ ppm;
MS: M$^+$ = 182 (3.5); m/e: 111 (6.5), 97 (4.5), 84 (54), 71 (5), 57 (100), 41 (25), 29 (14).
Die Verbindung weist eine interessante animalische Note auf.

Beispiel 2

Herstellung von 2,5,7,7-Tetramethyl-octanal

In einem Hydrierautoklaven wurden 250 g (1,37 mol) 2,5,7,7-Tetramethyl- 2-octenal gelöst in 500 ml Methanol in Gegenwart von 25 g eines 0,5 % Palladium/Aluminiumoxid-Katalysators bei 80°C zunächst bei einem Wasserstoffdruck von 25 bar, nach 2 bis 3 h bei 55 bar hydriert. Die gesamte Hydierzeit betrug 10 h. Nach Abfiltrieren des Katalysators wurden durch fraktionierte Destillation 245 g (1,33 mol) 2,5,7,7-Tetramethyl-octanal erhalten (Kp. = 68°C/0,3 mbar; $n_D^{25}$ = 1.4308), was einer Ausbeute von 96% der Theorie entspricht. Die Verbindung lag als Diastereomerengemisch vor.
IR: 2956, 1728, 1476, 1465, 1365 cm$^{-1}$;
$^1$H-NMR: 0,89 (9H, s), 0,92 (3H, d, 1-1.5 (9H, m), 1.7 (1H, m), 2.25 (1H, m), 9.15 (1H, s) δ ppm;
MS: M$^+$ = 184 (0.1); m/e: 151 (2.5), 142 (2.0); 128 (6.0), 126 (5.5), 110 (5.5), 95 (13.5), 81 (5), 71 (22), 57 (100), 41 (32).

Die Verbindung weist eine hochinteressante frische intensive Maiglöckchen-Note auf.

Beispiel 3

2-Ethyl-5,7,7-trimethyl-octanal

Die Herstellung von 2-Ethyl-5,7,7-trimethyl-octanal erfolgte aus 3,5,5-Trimethyl-hexanal und n-Butanal analog den Beispielen 1 und 2; sie lag als Diastereomerengemisch vor.
Kp. = 102°C/14 mbar; $n_D^{25}$ = 1.4339.
IR: 2557, 1728, 1476, 1464, 1365, 1247 cm$^{-1}$;
$^1$H-NMR: 0.88 (9H, s), 0.89 (3H, d),0.89 (3H, t), 0.95 - 1.7 (9H, m), 2.13 (1H, m), 9.63 (1H, s) δ ppm;
MS: M$^+$ = 198 (0,6); m/e: 142 (7), 126 (7.5), 109 (8), 95 (6,8), 83 (5), 72 (37), 57 (100), 41 (27), 29 (18).

Die Verbindung weist eine frische Blumennote auf.

Beispiel 4

2,4,5,7,7-Pentamethyl-octanal

Die Herstellung von 2,4,5,7,7-Pentamethyl-octanal erfolgte aus 2,3,5,5-Tetramethyl-hexanal (Aldehyd TMH/Firma Dragoco, Holzminden) und Propionaldehyd analog den Beispielen 1 und 2; die Verbindung lag als Diastereomerengemisch vor.

Kp. = 54°C/0.2 mbar; $n_D^{25}$ = 1.4398
IR: 2958, 1727, 1477, 1465, 1393, 1381, 1365, 1113 $cm^{-1}$
$^1$H-NMR: 0.78-0.9 (6H, m), 0.9 (9H, s), 0.9-1.8 (9H, m), 2,4 (1H, m), 9.58 und 9.63 (1H, s) δ ppm;
MS: $M^+$ = 198 (0.1); m/e: 140 (3.5), 109 (6.5), 99 (12), 85 (13), 75 (3.5), 71 (17), 57 (17), 43 (47).
Die Verbindung weist einen interessanten Blumenduft mit einer Calmus-Note auf.

<u>Anwendungsbeispiel</u>

Eine Parfüm-Grundzusammenstellung mit blumigem Charakter wurde hergestellt durch Vermischen der folgenden Bestandteile

| | | |
|---|---|---|
| Citronellol | 33,7 | g |
| Rhodinol P (Rhone-Poulenc) | 11,3 | g |
| Hydroxycitronellal (BASF) | 9,6 | g |
| Benzylbenzoat | 6,7 | g |
| Dimethylphthalat | 2,25 | g |
| Indol 10% | 2,25 | g |
| | 95,00 | g |
| 2,5,7,7-Tetramethyl-octanal | 5,00 | g |
| | 100,00 | g |

Der Zusatz von 2,5,7,7-Tetramethyl-octanal bewirkte eine Verstärkung des Duftes der Komposition natürlicher blumiger Frische.

**Patentansprüche**

1. Aliphatische Aldehyde der allgemeinen Formel I

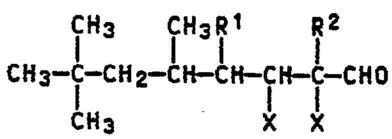

(I),

in der
$R^1$ für Wasserstoff oder eine Methylgruppe steht,
$R^2$ für einen geradkettigen oder verzweigten Alkylrest mit 1 bis 4 C-Atomen steht
und die beiden X jeweils für Wasserstoff stehen oder zusammen eine weitere CC-Bindung bedeuten.

2. 2,5,7,7-Tetramethyl-octanal.
3. 2,4,5,7,7-Pentamethyl-octanal.
4. 2-Ethyl-5,7,7-trimethyl-octanal.
5. 2,5,7,7-Tetramethyl-2-octen-1-al.
6. Verfahren zur Herstellung von aliphatischen Aldehyden der allgemeinen Formel I, <u>dadurch gekennzeichnet</u>, daß man einen Aldehyd der allgemeinen Formel II

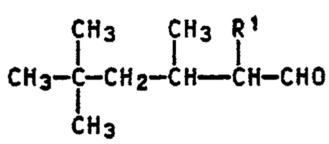

(II)

mit einem Aldehyd der allgemeinen Formel III

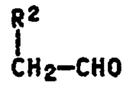

(III)

in denen $R^1$ und $R^2$ die oben angegebene Bedeutung haben in Gegenwart eines Aldolkondensationskatalysators zu dem substituierten Octenal der allgemeinen Formel I kondensiert und dieses ggf. partiell zu dem entsprechenden Octanal der Formel I hydriert bzw. reduziert.

7. Riechstoffkompositionen, enthaltend mindestens einen der aliphatischen Aldehyde nach den Ansprüchen 1 bis 5.

8. Riechstoffkompositionen, gekennzeichnet durch einen Gehalt von 1 bis 50 Gew.% an den aliphatischen Aldehyden der Formel I gemäß Anspruch 1.

9. Verwendung der Aldehyde der Formel I gemäß Anspruch 1 zur Verleihung, Verbesserung oder Modifizierung der Dufteigenschaften von Parfüms oder parfümierten Produkten.

## Claims

1. An aliphatic aldehyde of the formula I

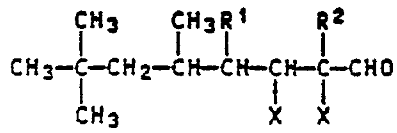

(I)

where
$R^1$ is hydrogen or methyl,
$R^2$ is a straight-chain or branched alkyl radical of 1 to 4 carbon atoms and the two radicals X are each hydrogen or together form a further C–C bond.

2. 2,5,7,7-Tetramethyloctanal.

3. 2,4,5,7,7-Pentamethyloctanal.

4. 2-Ethyl-5,7,7-trimethyloctanal.

5. 2,5,7,7-Tetramethyl-2-octen-1-al.

6. A process for the preparation of an aliphatic aldehyde of the formula I, wherein an aldehyde of the formula II

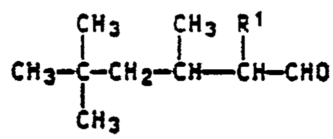

(II)

is condensed with an aldehyde of the formula III

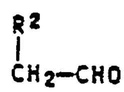

(III)

where $R^1$ and $R^2$ have the above meanings, in the presence of an aldol condensation catalyst to give the substituted octenal of the formula I, and, if required, the latter is partially hydrogenated or reduced to give the corresponding octanal of the formula I.

7. A scent composition containing one or more of the aliphatic aldehydes as claimed in any of claims 1 to 5.

8. A scent composition which contains from 1 to 50% by weight of the aliphatic aldehydes of the formula I as claimed in claim 1.

9. Use of an aldehyde of the formula I as claimed in claim 1 for imparting, improving or modifying the fragrance properties of perfumes or perfumed products.

## Revendications

1. Aldéhydes aliphatiques de formule générale I

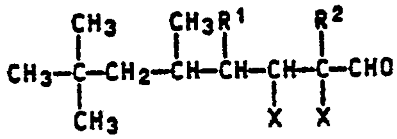

(I),

dans laquelle
$R^1$ représente un atome d'hydrogène ou un groupement méthyle,
$R^2$ représente un reste alkyle à chaîne droite ou ramifiée de 1 à 4 atomes de carbone et les deux X représentent chacun un atome d'hydrogène ou ensemble une autre liaison C–C.

2. 2,5,7,7-Tétraméthyl-octanal.

3. 2,4,5,7,7-Pentaméthyl-octanal.

4. 2-Ethyl-5,7,7-triméthyl-octanal.

5. 2,5,7,7-Tétraméthyl-2-octèn-1-al.

6. Procédé de préparation d'aldéhydes aliphatiques de formule générale I, caractérisé en ce qu'on condense un aldéhyde de formule générale (II)

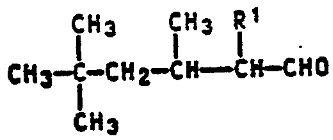

(II)

avec un aldéhyde de formule générale (III)

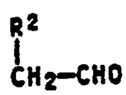

(III)

dans lesquelles $R^1$ et $R^2$ sont tels que définis ci-dessus, en présence d'un catalyseur de condensation aldolique, en l'octénal substitué de formule générale I et on réduit ou on hydrogène éventuellement celui-ci partiellement en l'octanal correspondant de formule I.

7. Compositions odorantes, contenant au moins un des aldéhydes aliphatiques selon les revendications 1 à 5.

8. Compositions odorantes, caractérisées par une teneur de 1 à 50% en poids d'un aldéhyde aliphatique de fomrule I selon la revendication 1.

9. Utilisation des aldéhydes de formule I selon la revendication 1, pour donner des propriétés odorantes, les améliorer ou les modifier à des parfums ou des produits parfumés.